Europäisches Patentamt

European Patent Office

Office européen des brevets

(19)

(11) Veröffentlichungsnummer: **0 141 955**
**A2**

(12) # EUROPÄISCHE PATENTANMELDUNG

(21) Anmeldenummer: 84110587.7

(51) Int. Cl.⁴: **D 04 B 15/60**

(22) Anmeldetag: 06.09.84

(30) Priorität: 16.09.83 DE 3333500

(71) Anmelder: Sulzer Morat GmbH, D-7024 Filderstadt 4 (DE)

(43) Veröffentlichungstag der Anmeldung: 22.05.85
Patentblatt 85/21

(72) Erfinder: Leins, Eberhard, Uhlberstrasse 66,
D-7024 Filderstadt 4 (DE)

(84) Benannte Vertragsstaaten: CH FR GB LI

(74) Vertreter: Freiherr von Schorlemer, Reinfried,
Brüder-Grimm-Platz 4, D-3500 Kassel (DE)

(54) **Fadenführer.**

(57) Die Erfindung betrifft einen Fadenführer für eine Strickmaschine mit wenigstens einer steuerbaren Fadenklemme und einem Einlegeorgan (19), dessen Auslaufende für den Faden (16) als offener Kanal (76) ausgebildet ist. Zur Vereinfachung des Einlegens bzw. Auslegens von Fäden sind die Fadenklemme (109) und das Einlegeorgan (19) derart relativ zueinander beweglich angeordnet, dass der Faden von der Fadenklemme (109) selbst wahlweise in den Kanal (76) einlegbar oder aus diesem herausnehmbar ist.

## Fadenführer

Die Erfindung betrifft einen Fadenführer für eine Strickmaschine mit einem Einlegeorgan, das an seinem Auslaufende
für den Faden als offener Kanal ausgebildet ist, und mit
wenigstens einer steuerbaren Fadenklemme.

Fadenführer dieser Art (GB-PS 1 502 370, GB-OS 20 99 464)
dienen dazu, einen Faden für eine bestimmte Zeitlang den
Nadeln einer Strickmaschine zuzuführen und danach wieder
zu verklemmen. Zu diesem Zweck sind die Fadenführer in der
Regel mit wenigstens einem Schwenkhebel versehen, der zum
Einlegen und Auslegen des Fadens zwischen dem häufig auch
als "Nüßchen" bezeichneten Einlegeorgan und der Fadenklemme
hin- und hergeschwenkt wird, während die Fadenklemme und
das Einlegeorgan im wesentlichen ortsfest angeordnet sind.
Dadurch ergeben sich Schwierigkeiten bei der automatischen
Steuerung, insbesondere wenn die Fadenführer beispielsweise
in Verbindung mit speziellen Flachstrickmaschinen
(US-PS 4 364 246) als Ganzes an einem Nadelbett entlang geführt werden müssen. Handelt es sich dabei um einen Fadenführer, mit dem mehrere Fäden wahlweise eingelegt werden
sollen, sind entsprechend viele Schwenkhebel und Fadenklemmen erforderlich. Dadurch wird der Fadenführer zusätzlich
raumaufwendig.

Der Erfindung liegt daher die Aufgabe zugrunde, den Fadenführer der eingangs bezeichneten Gattung so auszubilden,
daß er auf einfache Weise gesteuert werden kann und den
Zugang zu den Strickwerkzeugen der Strickmaschine möglichst
wenig behindert.

Zur Lösung dieser Aufgabe ist der eingangs bezeichnete
Fadenführer dadurch gekennzeichnet, daß das Einlegeorgan
und die Fadenklemme derart relativ zueinander beweglich
angeordnet sind, daß der Faden von der Fadenklemme selbst
wahlweise in den Kanal einlegbar oder aus diesem herausnehmbar
ist.

- 2 -

Die Erfindung bringt den Vorteil mit sich, daß keine zusätzlichen Schwenkhebel od. dgl. benötigt werden, um den Faden
in das Einlegeorgan einzulegen. Dadurch wird der Raumbedarf
des Fadenführers verringert und eine einfache Steuerung auch
für den Fall ermöglicht, daß der Fadenführer als Ganzes an
einem Nadelbett entlang geführt werden muß.

Der erfindungsgemäße Fadenführer eignet sich insbesondere
für solche Strickmaschinen (US-PS 4 364 246), bei denen die
Fadenführer auf einer Endlosbahn umlaufen.

Die Erfindung wird nachfolgend in Verbindung mit der beiliegenden Zeichnung an einem Ausführungsbeispiel näher erläutert. Es zeigen:

Fig. 1 in perspektivischer, schematischer Darstellung eine Strickmaschine mit erfindungsgemäßen Fadenführern;

Fig. 2 die Seitenansicht eines erfindungsgemäßen Fadenführers senkrecht zu seiner Transportrichtung;

Fig. 3 eine teilweise geschnittene Seitenansicht des Fadenführers nach Fig. 2, wobei die Schnittlinie durch seine parallel zur Transportrichtung verlaufende Mittelebene gelegt ist und die Fadenklemmen in einer anderen Stellung dargestellt sind;

Fig. 4 eine Seitenansicht des Fadenführers entsprechend Fig. 2 in einer anderen Stellung der Fadenklemmen;

Fig. 5 eine teilweise längs der Linie V-V der Fig. 3 geschnittene Vorderansicht des Fadenführers nach Fig. 2;

Fig. 6 eine teilweise längs der Linie VI-VI der Fig. 3 geschnittene Vorderansicht des Fadenführers nach Fig. 2;

Fig. 7 eine Seitenansicht des Fadenführers nach Fig. 2 in einer dritten Stellung der Fadenklemmen;

Fig. 8 eine teilweise geschnittene Draufsicht längs der Linie VIII-VIII der Fig. 2;

Fig. 9 bis 11 einen Steuerhebel des erfindungsgemäßen Fadenführers in einer Seitenansicht, Vorderansicht und Draufsicht;

Fig. 12 bis 14 ein Einlegeorgan des erfindungsgemäßen Fadenführers in einer Vorderansicht, Seitenansicht und Draufsicht;

0141955

Fig. 15 und 16 je einen vergrößerten Schnitt durch eine
Trommel des erfindungsgemäßen Fadenführers längs
der Linie VI-VI der Fig. 3;

Fig. 17 und 18 die Draufsicht und eine Seitenansicht eines
Arretierschiebers des Fadenführers nach Fig. 2;

Fig. 19 und 20 die Vorderansicht der Schaltschieber und
der Klemmfeder einer Fadenklemme des erfindungsgemäßen Fadenführers zur Darstellung der Klemm- und
Offenstellung;

Fig. 21 eine Draufsicht auf die Teile nach Fig. 19 und 20;

Fig. 22 und 23 je eine Seitenansicht der beiden Schaltschieber nach Fig. 19 und 20;

Fig. 24 eine Draufsicht auf die Klemmfeder nach Fig. 19
und 20;

Fig. 25 und 26 Draufsichten auf Kodierscheiben des erfindungsgemäßen Fadenführers in vergrößerter Darstellung; und

Fig. 27 bzw. 28 schematisch die zur Steuerung des erfindungsgemäßen Fadenführers benötigten Kurven und Nocken am
Anfang bzw. Ende des Arbeitsabschnitts einer mit dem
erfindungsgemäßen Fadenführer ausgerichteten Strickmaschine.

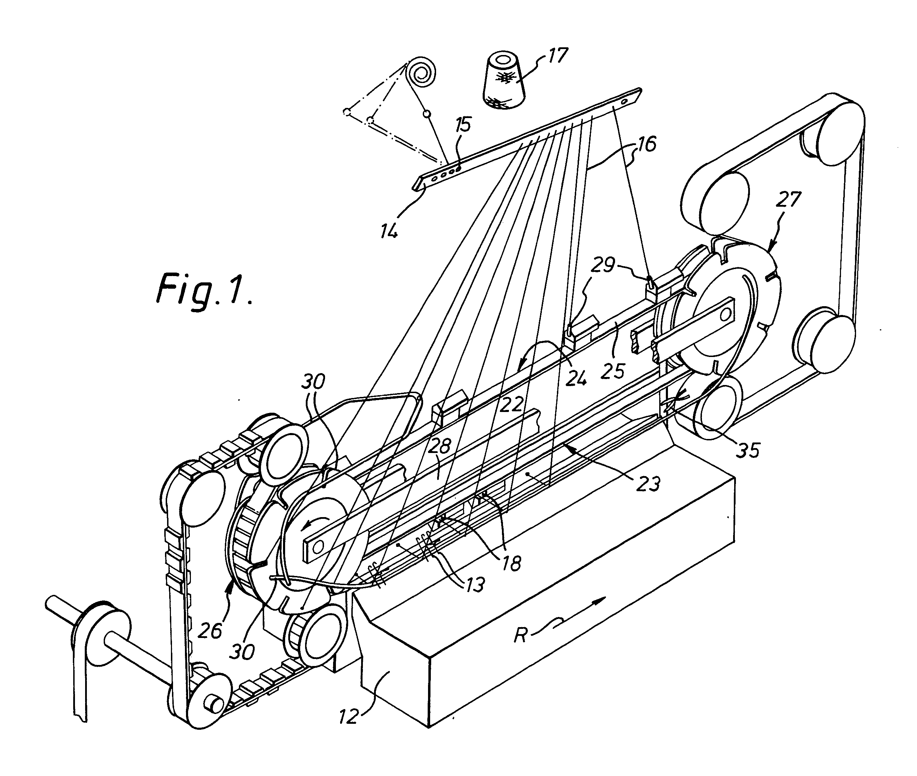

In Fig. 1 ist eine bekannte Flachstrickmaschine mit zwei
Nadelbetten 12 dargestellt, in deren Nuten in bekannter
Weise Nadeln 13, z.B. Zungennadeln, längsverschieblich
geführt sind. Oberhalb der Maschine ist ein Ösenträger 14
vorgesehen, durch dessen Ösen 15 mehrere Fäden 16 von stationären Vorratsspulen 17 zu einer Vielzahl von Fadenführern 18 mit Einlegeorganen 19 geführt sind. Zum Transport
der Fadenführer 18 ist eine endlose, 0-förmige Umlaufbahn 22

0141955

vorgesehen, die einen oberhalb des Arbeitsabschnitts der
Einlegeorgane 19 und parallel dazu angeordneten Abschnitt 23
und einen Abschnitt 24 aufweist. Auf dem Abschnitt 23 werden die Fadenführer 18 mit nach unten gerichteten Einlegeorganen 19 in Richtung eines Pfeils R geführt, so daß die
Fäden 16 in die Nadeln 13 eingelegt werden können. Nach Erreichen des in Fig. 1 rechts liegenden Gestrickendes werden
die Fadenführer 18 dagegen mit nach oben weisenden Einlegeorganen 19 längs des Abschnitts 24 zu dem in Fig. 1 links
befindlichen Gestrickanfang zurücktransportiert, wobei den
Nadeln 13 keine Fäden zugeführt und die Einlegeorgane 19
längs eines Rückführabschnitts bewegt werden.

Die Umlaufbahn 22 ist durch ein endloses, flexibles Transportband 25 gebildet, an dem die Fadenführer 18 befestigt
sind und das durch zwei Umlenkräder 26 und 27 festgelegt
ist, deren Achsen an den Enden je einer starren Schiene 28
gelagert sind. Um zu erreichen, daß sich die Fäden 16 beim
wiederholten Umlaufen der Fadenführer 18 nicht verseilen,
werden sie mit Hilfe je eines schwenkbar an ihnen befestigten
Schwenkhebels 29 und mehrerer Leitschienen 30 abwechselnd
auf der einen und auf der anderen Breitseite der Umlaufbahn 22
angeordnet. Im übrigen handelt es sich vorzugsweise um eine
Flachstrickmaschine mit mehreren, auf einer endlosen Umlaufbahn umlaufenden Schlitten oder um eine Flachstrick-
                                              oder Kurbeln
maschine, deren Nadeln durch exzentrische Kurvenscheiben/angetrieben werden, die mit Winkelversatz auf einer rotierenden Welle befestigt sind. Weitere Einzelheiten lassen sich
den DE-OS'en 25 31 762, 27 01 652 und 30 03 570 entnehmen,
auf die hiermit zur Vermeidung weiterer Beschreibungen ausdrücklich hingewiesen wird.

Die Nadeln 13 definieren in ihrer ausgetriebenen Stellung
einen parallel zu den Oberkanten der Nadelbetten 12 verlaufenden Arbeitsabschnitt, der als gerade Linie zu verstehen
ist, die dicht oberhalb des von den Nadeln 13 gebildeten
Nadelkreuzes und parallel zu diesem angeordnet ist und auf
der sich die Einlegeorgane 19 bewegen müssen, damit die Fäden 16 von den Nadeln 13 erfaßt und zu Maschen verarbeitet werden können. Dabei ist bei der Beschreibung des Fadenführers 18 angenommen, daß die die Nadeln 13 führenden Abschnitte der Nadelbetten 12 unter einem Winkel von je etwa 45° zum horizontalen Fußboden angeordnet sind und parallele sowie horizontale Oberkanten aufweisen. Die nachfolgenden Angaben betreffend die Lage, Anordnung oder Stellung verschiedener Teile des Fadenführers 18 beziehen sich daher auf diejenige Stellung, die dieser beim Durchgang des Einlegeorgans 19 durch den Abschnitt 23 einer solchen Strickmaschine einnimmt. Die dabei in Bewegungsrichtung vorn (in Fig. 1 rechts) bzw. hinten (in Fig. 1 links) liegenden Teile werden durchweg als "vordere" bzw. "hintere" Teile bezeichnet. Entsprechend werden stets die dem Nadelkreuz nahen Teile als "untere", die dem Nadelkreuz fernen Teile dagegen als "obere" Teile bezeichnet. Schließlich werden als "rechte" bzw. "linke" Teile solche Teile bezeichnet, die beim Durchgang der Einlegeorgane 19 durch den Arbeitsabschnitt rechts bzw. links von der durch das Nadelkreuz verlaufenden Vertikalebene angeordnet sind. Wird der erfindungsgemäße Fadenführer auf eine Maschine mit einer anderen Anordnung der Nadelbetten bzw. der Nadeln angewendet, dann müßten diese Bezeichnungen entsprechend geändert werden.

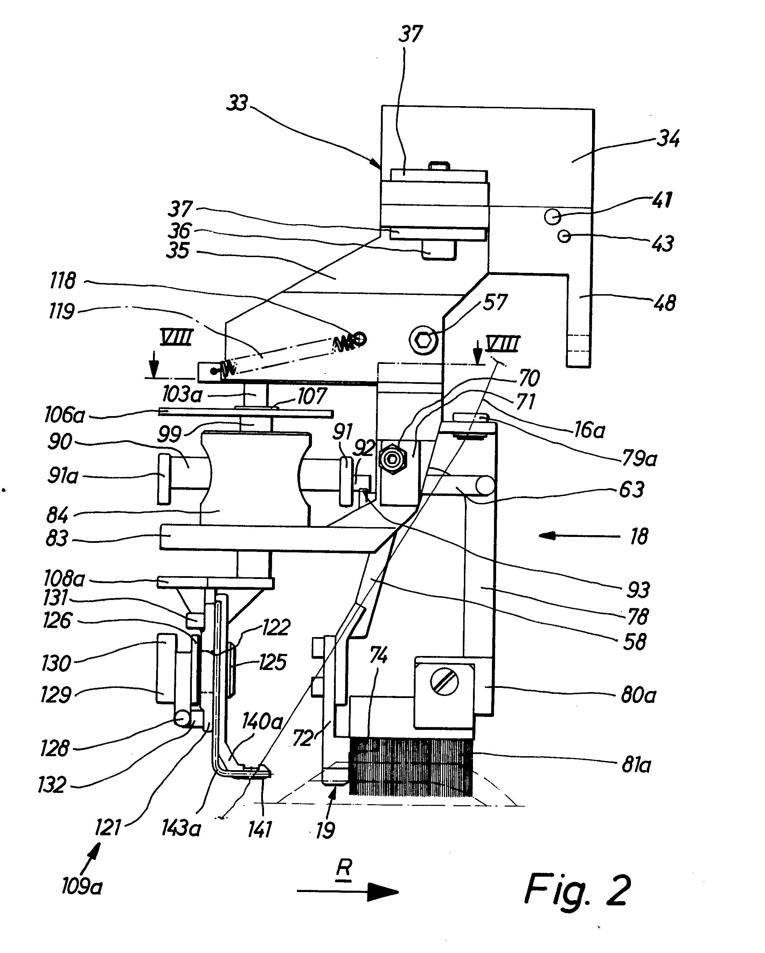

Anhand der Fig. 2 bis 6 wird nun die erfindungsgemäße Ausgestaltung des Fadenführers 18 beschrieben. Dabei ist angenommen, daß sich irgendein Fadenführer 18, solange sich sein Einlegeorgan 19 innerhalb des Arbeitsabschnitts befindet, stets in Richtung des Pfeils R, d.h. in der Zeichnung von links nach rechts bewegt.

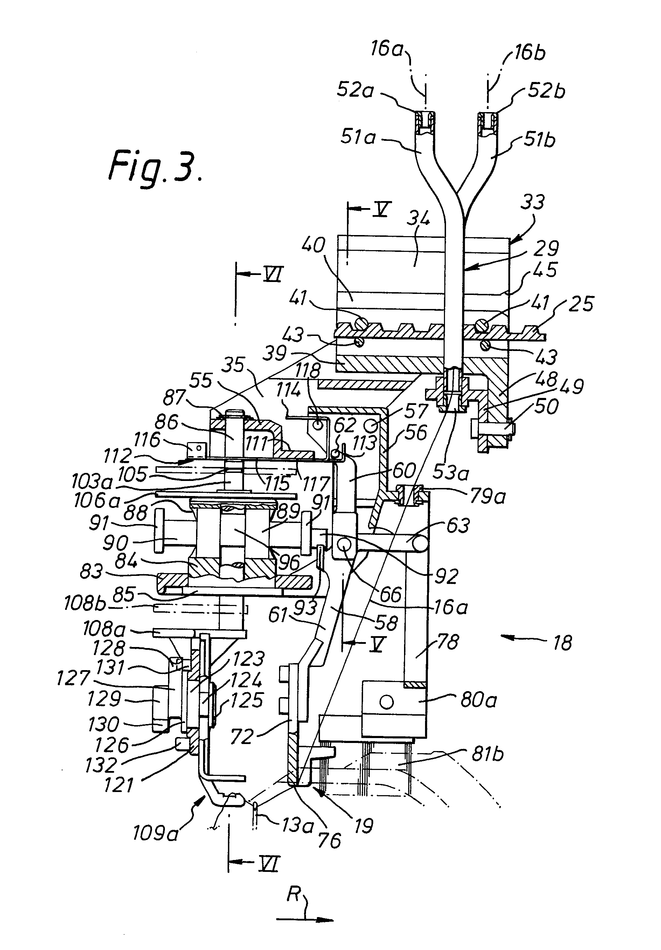

Zur Übertragung der Bewegung des nach Fig. 3 als Zahnriemen ausgebildeten Transportbandes 25 auf den Fadenführer 18 dient ein Führungsteil 33, das aus einem Oberteil 34 und einem Unterteil 35 besteht. Das Oberteil 34 und das Unterteil 35 weisen flanschförmige Verbreiterungen auf, die von Befestigungsschrauben 36 durchragt und ggf.

mit Metallscheiben 37 belegt sind, um bei ihrer Herstellung
aus Kunststoff Eindrückungen zu vermeiden.

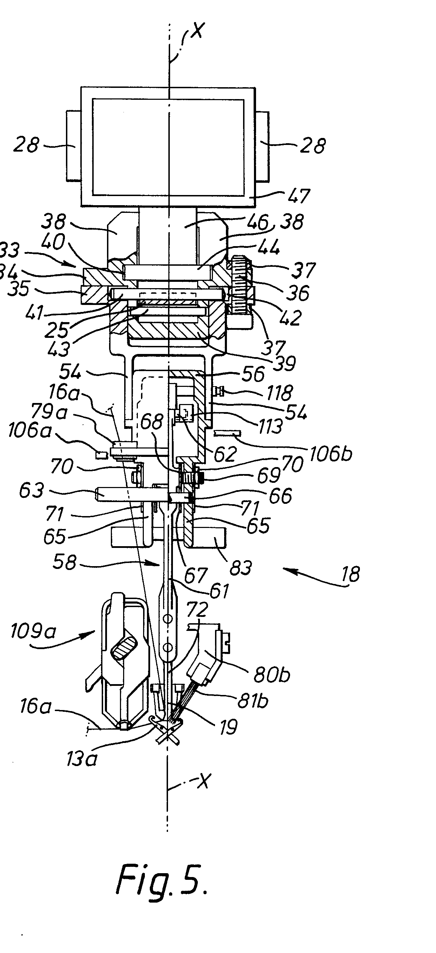

Das Oberteil 34 besitzt einen U-förmigen Querschnitt (Fig. 5)
und besteht aus zwei seitlichen, parallelen Führungsstegen 38,
deren untere Enden durch einen Quersteg 39 verbunden sind,
wobei beide Führungsstege 38 mit mittleren Führungsnuten 40
versehen sind, unterhalb von denen das Transportband 25 angeordnet ist. Die Kopplung des Fadenführers 18 mit dem Transportband 25 erfolgt mit Hilfe von Mitnehmerstiften 41, die
in den Führungsstegen 38 ausgebildete Bohrungen durchragen
und sich jeweils so zwischen zwei Zähne des Transportbandes
25 legen, daß kein Spiel in Transportrichtung auftreten kann.
Um zu vermeiden, daß die mittels der Befestigungsschraube 36
hergestellte Schraubverbindung  zu stark belastet wird, ragen
die Enden wenigstens eines Mitnehmerstifts 41 auch in je
eine im Unterteil 35 ausgebildete Ausnehmung 42 (Fig. 5),
wodurch das  den Fadenführer 18 tragende Unterteil 35 direkt
an das Transportband 25 gekoppelt wird. Um zu vermeiden, daß
die Mitnehmerstifte 41 die Zähne des Transportbandes 25 überlaufen können, sind Haltestifte 43 vorgesehen, die durch in
den Führungsstegen 38 ausgebildete Bohrungen ragen und sich
der von Zähnen freien Breitseite des Transportbandes 25 anlegen.

Das Oberteil 34 gleitet innerhalb des Arbeitsabschnitts
auf einer Führungsschiene 44 (Fig. 5), die in die Führungsnuten 40 eintritt, welche dazu mit je einem Einführungstrichter 45 (Fig. 3) versehen sind und die Fadenführer 18 innerhalb des  Abschnitts  23  genau parallel zum Nadelkreuz
führen. Die Führungsschiene 44 ist an einem Führungsbalken 46
befestigt, der am Anfang des  Abschnitts  23  zwischen die
beiden Führungsstege 38 tritt und diese seitlich führt, so
daß die Fadenführer 18 keine Kippbewegungen ausführen können.
Hierzu können auch die Führungsstege 38 an ihren Vorderenden
mit Einführungsschrägen versehen sein. Der Führungsbalken 46
ist an einer Konsole 47 aufgehängt, die beispielsweise an den
starren Schienen 29 (Fig. 1) befestigt ist.

An seinem Vorderende weist das Oberteil 34 einen Arm 48 auf,
an dem ein Winkelstück 49 (Fig. 3) mittels eines Zapfens 50
schwenkbar befestigt ist. Das Winkelstück 49 trägt den Schwenkhebel 29, der zwei parallele Rohre 51a,b mit je einer Einlauföse 52a,b und Auslauföse 53a,b für je einen schematisch angedeuteten Faden 16a,b aufweist.

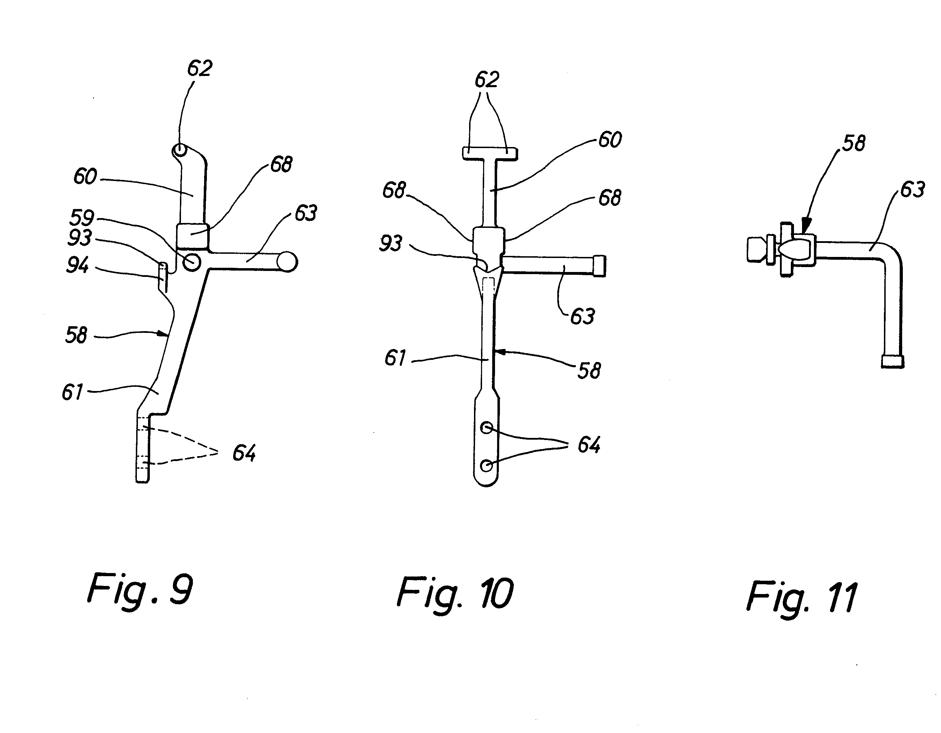

Das Unterteil 35 weist zwei Seitenwände 54 (Fig. 5) und
eine Stützplatte 55 (Fig. 3) auf. Zwischen den Seitenwänden 54 ist ein Gehäuse 56 mittels Schrauben 57 befestigt.
In diesem Gehäuse 56 ist ein Steuerhebel 58 schwenkbar gelagert, der in Fig. 9-11 in drei Ansichten dargestellt ist.
Der Steuerhebel 58 enthält in einem mittleren Teil eine Boh -
rung 59 zur schwenkbaren Lagerung und oberhalb bzw. unterhalb
dieser Bohrung 59 je einen Arm 60 bzw. 61. Der obere Arm 60
ist an seinem Ende mit zwei seitlich wegragenden, zur Achse
der Bohrung 59 parallelen Haltezapfen 62 versehen. In seinem
mittleren Teil weist der Steuerhebel 53 einen seitlichen
Schwenkbügel 63 auf, der zunächst senkrecht zur Achse der
Bohrung 59 verläuft und dann derart gebogen ist, daß die
Mittelachse seines freien Endes im wesentlichen parallel
zu der Achse der Bohrung 59 angeordnet ist. Der untere Arm
61 des Steuerhebels 58 weist an seinem freien Ende zwei Gewindebohrungen 64 auf.

Gemäß Fig. 5 ist der Steuerhebel 58 zwischen zwei Seitenwänden 65 des Gehäuses 56 auf einem Drehbolzen 66 schwenkbar
gelagert, der Bohrungen dieser Seitenwände 65 und die Bohrung 59 durchragt. Zur seitlichen Justierung des Steuerhebels 58 auf dem Drehbolzen 66 dienen Lagerbleche 67, die
ebenfalls vom Drehbolzen 66 durchragt werden und sich an
oberhalb der Bohrung 59 ausgebildeten Lagerflächen 68 des
Steuerhebels 58 abstützen, die gemäß Fig. 5,9 und 10 an
einem verbreiterten Teil des Arms 60 angebracht sind. Im
Bereich der Lagerflächen 68 stützt sich je eine Einstellschraube
69 auf der Außenseite der Lagerbleche 67 ab, wobei jede Einstellschraube 69 eine Gewindebohrung in der zugehörigen Seitenwand 65 durchragt und außen mit einer Kontermutter 70 versehen ist.
Zwischen der Kontermutter 70 und der Seitenwand 65 ist ein
weiteres, von der Einstellschraube 69 durchragtes Lagerblech
71 angeordnet, das die den Drehbolzen 66 aufnehmenden Bohrungen der Seitenwände 65 abdeckt. Alle Lagerbleche 67 und 71
stützen sich außerdem derart an vorspringenden Rändern der
Seitenwände 65 ab (Fig. 2), daß sie weitgehend undrehbar
gehalten sind. Diese Lagerung hat   zur  Folge,
daß der Steuerhebel mittels der Einstellschraube 69 in Achsrichtung des Drehzapfens 66 spielfrei einjustiert werden kann,
um Herstellungstoleranzen auszugleichen, daß ferner die inneren Lagerbleche 67 nur an den Lagerflächen 68 anliegen und
dadurch eine gute Gleitlagerung bewirken und daß sich nach
erfolgter Justierung die Kontermuttern 70 nicht lösen können.

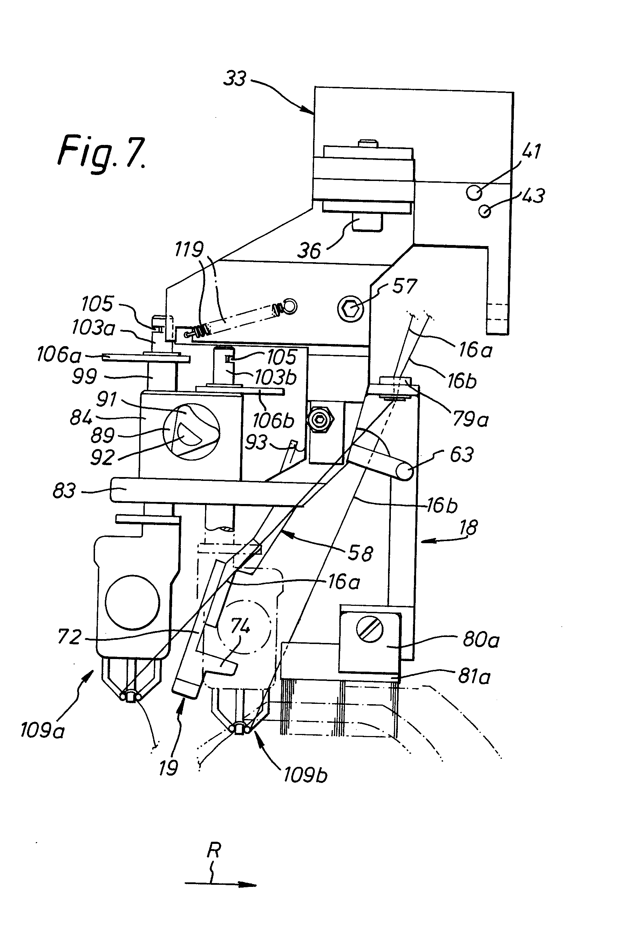

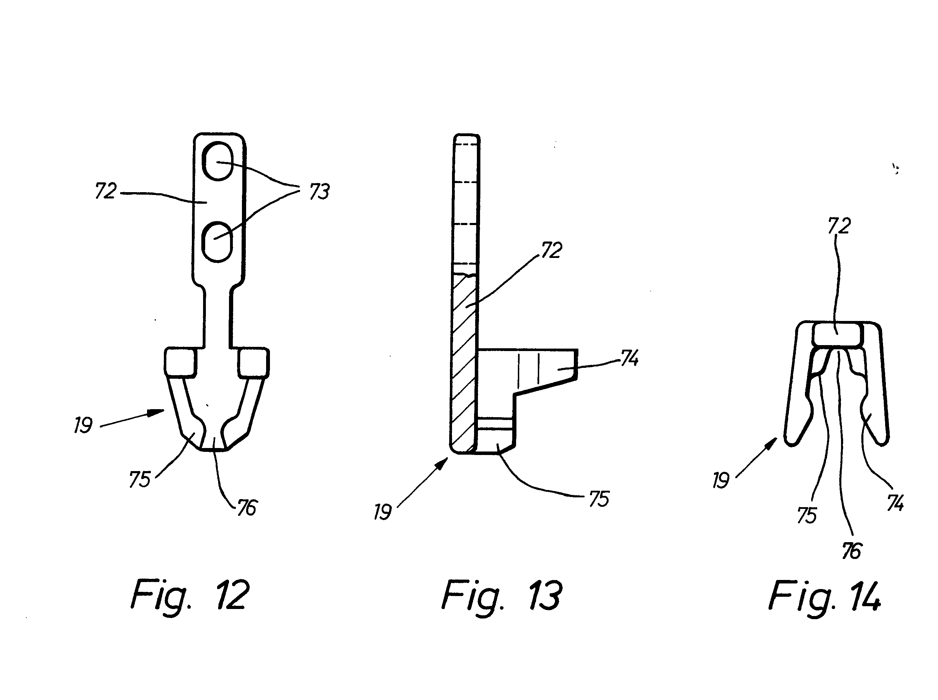

Am Arm 61 des Steuerhebels 58 ist mit Schrauben, die in die
Gewindebohrungen 64 eingedreht sind, ein nach unten ragender Steg 72 befestigt, der an seinem unteren Ende das Einlegeorgan 19 des Fadenführers 18 trägt und aufgrund von die
Befestigungsschrauben aufnehmenden Langlöchern 73 in seiner
Höhenlage exakt eingestellt werden kann. Das Einlegeorgan 19,
das in Fig. 12 bis 14 in drei Ansichten dargestellt ist, besitzt an seinem Vorderende eine nach Art eines Einlauftrichters ausgebildete Gabel 74 und in seinem Boden 75 einen keilförmigen, nach oben und unten offenen, schlitzartigen Kanal 76,
dessen Mittelachse in der aus Fig. 3 ersichtlichen Normalstellung
des Steuerhebels 58 genau in einer Mittelebene X des Fadenführers 18 und gemäß Fig. 7 dicht oberhalb des Nadelkreuzes angeordnet ist, sich wie die Gabel 74 nach vorn öffnet und im Arbeitsbereich geführt wird.

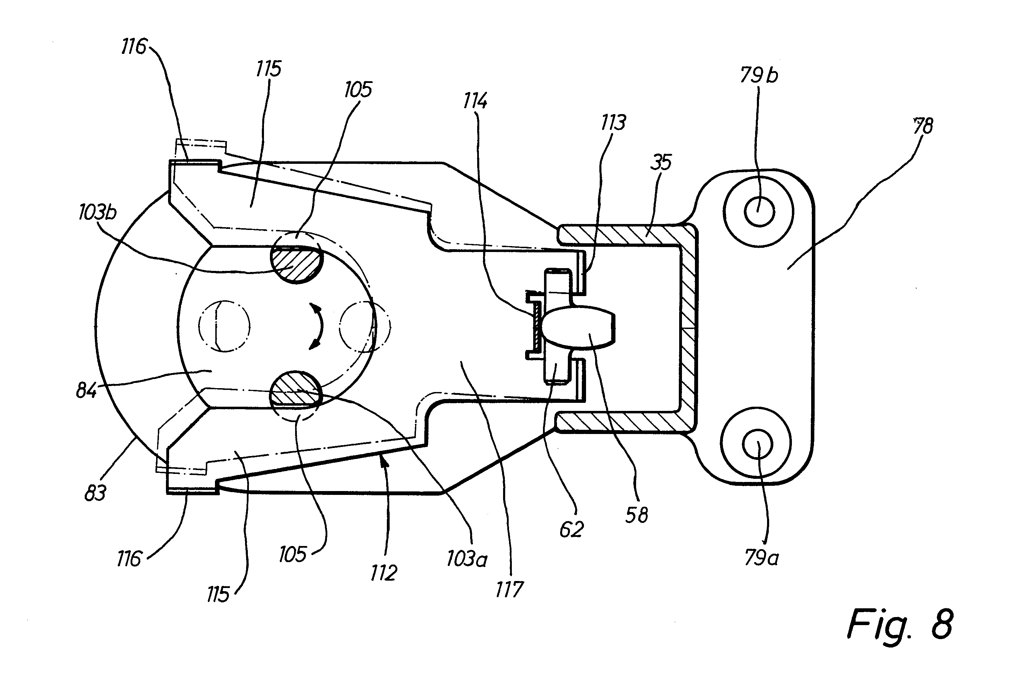

Am Vorderende des Gehäuses 56 ist ein vertikal angeordneter, nach unten ragender Tragarm 78 befestigt, der an seinem oberen Ende zwei Fadenösen 79a,b (Fig. 8), an seinem unteren
Ende dagegen zwei Halterungen 80a,b zur Befestigung je einer
die Nadelzungen öffnenden oder offen haltenden Bürste 81a,b trägt.
Diese Bürste 81a,b läuft dem Fadenführer 18 voraus und dient insbesondere dazu, die geöffneten Nadelzungen abzudecken und offen zu
halten. In Fig. 2 bis 4 und 7 sind jeweils nur die Teile 79a,80a
und 81a sichtbar, während Fig. 5 die Teile 80b und 81b zeigt, wobei die mit einem "a" versehenen Teile jeweils rechts, die mit einem "b" versehenen Teile dagegen links von der Mittelebene X des Fadenführers angeordnet sind.

An einem unteren Teil des Gehäuses 56 ist eine horizontale, nach innen ragende Tragplatte 83 vorgesehen. In einer Mittelbohrung der Tragplatte 83 ist eine Trommel 84 mit vertikaler Drehachse drehbar gelagert, die sich mit einem unteren, verbreiterten Rand 85 (Fig. 3) an die Tragplatte 83 legt und an ihrer Oberseite eine koaxiale, zylindrische Stange 86 trägt, die in einer Bohrung der Stützplatte 55 des Unterteils 35 drehbar gelagert und auf der von der Trommel 84 abgewandten Seite mit einem Sprengring 87 axial festgelegt ist. Die Trommel 84 weist eine quer zu ihrer Drehachse verlaufende Lagerbohrung 88 zur drehbaren Lagerung einer Welle 89 auf, an deren beiden Enden über Zwischenstücke 90 herzförmige Steuerscheiben 91 befestigt sind. An der Vorderseite der in Fig. 3 rechts liegenden Steuerscheibe 91 ist noch eine halbkreisförmige Sperrnase 92 angebracht. Dabei ist die Anordnung so getroffen, daß die Sperrnase 92 bei der aus Fig. 3 ersichtlichen Drehstellung der Trommel 84, bei der sowohl die Achse der Welle 89 als auch die Drehachse der Trommel 84 in der Mittelebene X liegen, in einer Aufnahme 93 ruht, die an einem rückwärtigen Vorsprung 94 des Steuerhebels 58 ausgebildet ist, dessen Schwenkachse vorzugsweise senkrecht zur Mittelebene X verläuft.

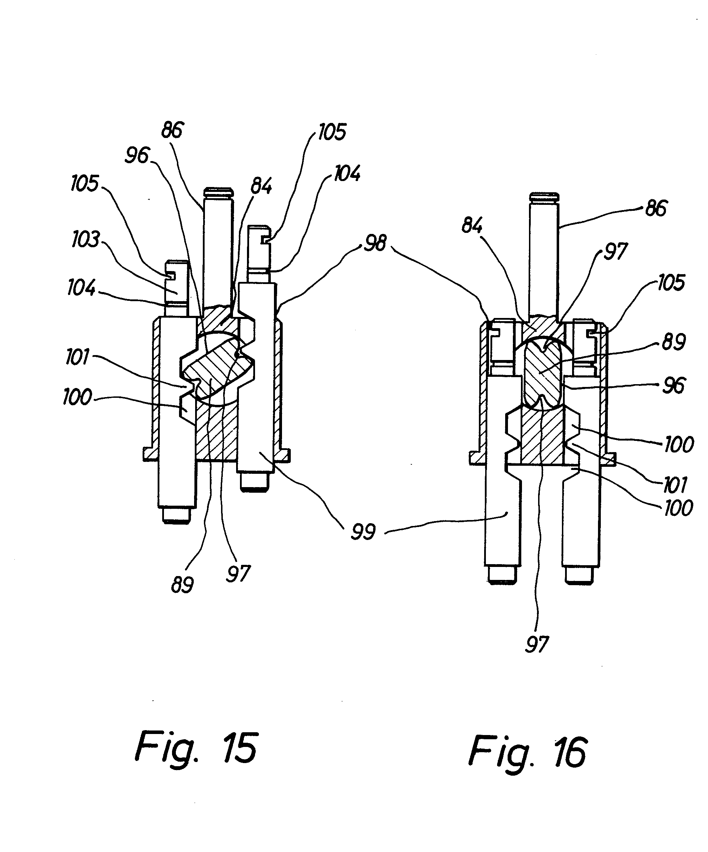

Gemäß Fig. 3, 15 und 16 ist die Welle 89 in ihrem Mittelteil und beidseitig ihrer Mittelachse mit einer Querschnittsverminderung 96 und je einer radial außen liegenden Zahnlücke 97 versehen, die dem Mittelstück der Welle 89 die Form eines Ritzels gibt. Die Trommel 84 weist ferner zwei zur Trommelachse parallele Längsbohrungen 98 auf, deren Achsen parallel zueinander und symmetrisch zu beiden Seiten der Stange 86 angeordnet sind. In den Längsbohrungen 98 ist je eine zylindrische Zahnstange 99 verschiebbar gelagert. Jede Zahnstange 99 ist in einem Mittelabschnitt mit zwei Aussparungen 100 versehen, die je einen Zahn 101 stehen lassen. Dabei ist die Anordnung so getroffen, daß die beiden Längsbohrungen 98 entsprechend Fig. 15 und 16 etwas in den von der Lagerbohrung 88 ausgesparten Hohlraum hineinreichen und jede Zahnstange 99 die Lagerbohrung 88 an deren Rändern teilweise durchragt.

Die Zahnstangen 99 werden bei der aus Fig. 16 ersichtlichen Drehstellung der Welle 89 von unten in die Längsbohrungen 98 eingeführt,wobei die Querschnittsverminderungen 96 die Längsbohrungen 98 freigeben. Wenn die Zahnstangen 99 etwa die aus Fig. 15 ersichtliche Stellung einnehmen, wird die Welle 89 derart gedreht, daß die jetzt in die Lagerbohrung 88 ragenden Zähne 101 in die Zahnlücken 97 eintreten.

An den oberen Enden weisen die Zahnstangen 99 koaxiale, jedoch nur etwa halbzylindrische Ansätze 103a,b von verringertem Querschnitt auf, die in einem unteren Bereich eine Ringnut 104 und an ihren oberen Enden eine seitliche Quernut 105 aufweisen. Auf beide Ansätze 103 ist je eine Kodierscheibe 106a,b aufgezogen, die eine entsprechende halbkreisförmige Mittelbohrung aufweist und daher undrehbar auf den Ansätzen gehalten ist. Die Kodierscheiben 106 werden nach dem Einführen der Zahnstangen 99 in die Längsbohrung 98 von oben her aufgeschoben, bis sie sich auf den oberen Schultern der Zahnstangen 99 abstützen, und dann mit je einem in die Ringnuten 104 gedrückten Sprengring 107 axial gesichert. An den unteren Enden der Zahnstangen 99 ist je ein Haltekörper 108a,b für eine Fadenklemme 109a,b befestigt. Dabei ist die Anordnung so getroffen, daß die Verschiebbarkeit der Zahnstangen nach oben und unten jeweils durch die auf die Oberseite der Trommel 84 stoßende Kodierscheibe 106 begrenzt ist und nach erfolgter Montage die Zähne 101 stets in den zugehörigen Zahnlücken 97 verbleiben. Durch Drehung der Welle 84 in der einen oder anderen Richtung lassen sich somit die Zahnstangen 99 auf- und abbewegen, wobei beide Zahnstangen 99 stets gleichzeitig, aber in entgegengesetzte Richtungen bewegt werden.

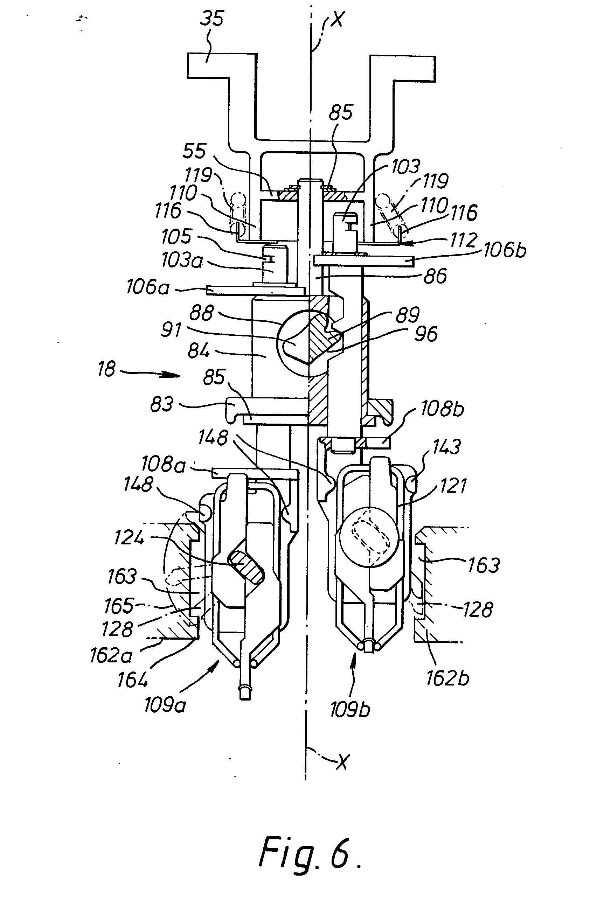

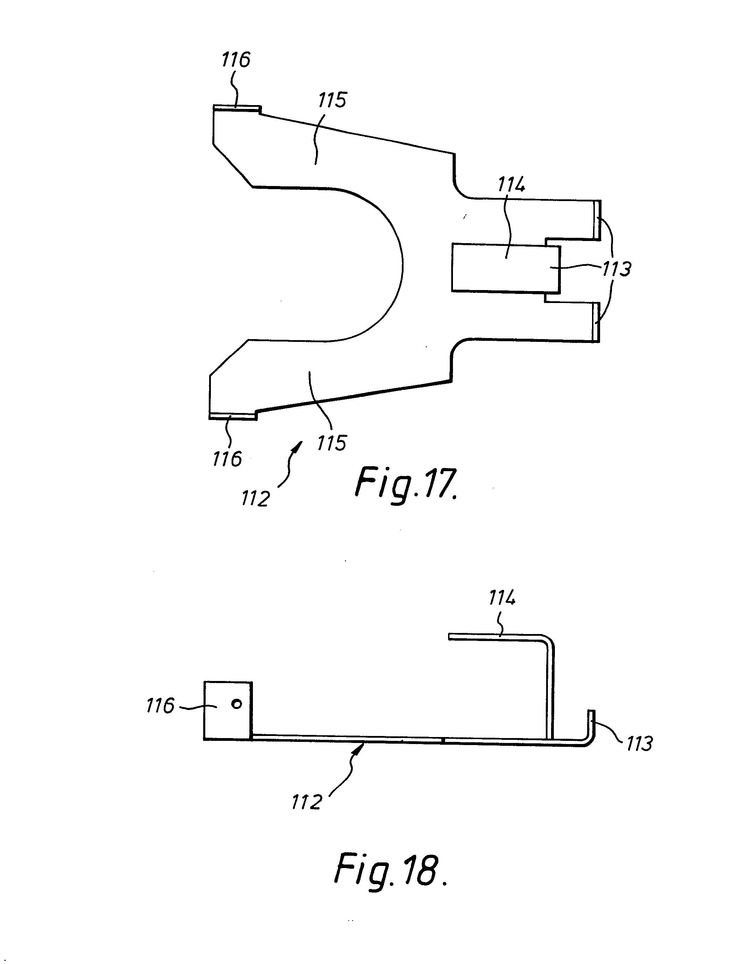

Gemäß Fig. 3 und 6 weist das Unterteil 35 zwei von der Stützplatte 55 U-förmig nach unten ragende Stege 110 auf, deren
Unterkanten zusammen mit der dazu parallelen Unterseite einer mit der Stützplatte 55 verbundenen Bodenplatte 111 als
Anschlag- und Gleitflächen für einen Arretierschieber 112
dienen. Dieser Arretierschieber 112 ist in Fig. 17 und 18
in zwei Ansichten dargestellt. Er besteht aus einem dünnen
Blechteil, das an seinem Vorderende zwei um ca. 90° nach oben
umgebogene Lappen 113 aufweist, die zu beiden Seiten einer
um ca. 180° nach oben umgebogenen Lagerzunge 114 angeordnet sind. An seinem rückwärtigen Ende ist der Arretierschieber 112 mit einer mittleren Aussparung versehen, die zwei
Gabelarme 115 einer nach hinten offenen Gabel stehen
läßt. Der Abstand der Gabelarme 115 ist etwa gleich dem Abstand der Böden der beiden Quernuten 105 in den Ansätzen 103.
Außerdem sind beide Gabelarme 115 durch einen Mittelabschnitt
117 mit den Lappen 113 und der Lagerzunge 114 verbunden, der
auf der den Gabelarmen 115 zugewandten Seite längs eines Kreisbogens verläuft, dessen Radius der Hälfte dieses Abstandes entspricht. An den Hinterenden der Gabelarme sind mit Bohrungen
versehene, um ca. 90° nach oben gebogene Laschen 116 vorgesehen.

Gemäß Fig. 3 ist die Anorndung so getroffen, daß der Arretierschieber 112 mit seinen Gabelarmen 115 den Stegen 110 und mit
seinem Zwischenstück 117 der Bodenplatte 111 anliegt und dabei gleichzeitig die Laschen 113 je einen Haltezapfen 62 des
Steuerhebels 58 umgreifen, während die Lagerzunge 114 einen
Stift 118 umgreift und auf diesem abgestützt ist, der die
Seitenwände des Unterteils 35 und des Gehäuses 56 durchragt.
Der Stift 118 dient dabei einerseits zur zusätzlichen Lagerfixierung des Gehäuses 56 am Unterteil 35, und andererseits mit
seinem aus dem Unterteil 34 herausragenden Ende als Aufhängung
für die einen Enden je einer Zugfeder 119, deren andere Enden
in die Bohrungen der Laschen 116 eingehängt sind (Fig. 2).
Die Zugfedern 119 ziehen den Arretierschieber 112 gegen die
Stege 110 bzw. die Bodenplatte 111 und spannen über die zwischen den Lappen 113 und der Lagerzunge 114 liegenden Haltezapfen 62 gleichzeitig den Steuerhebel 58 bei Blick auf
Fig. 3 im Uhrzeigersinn vor.

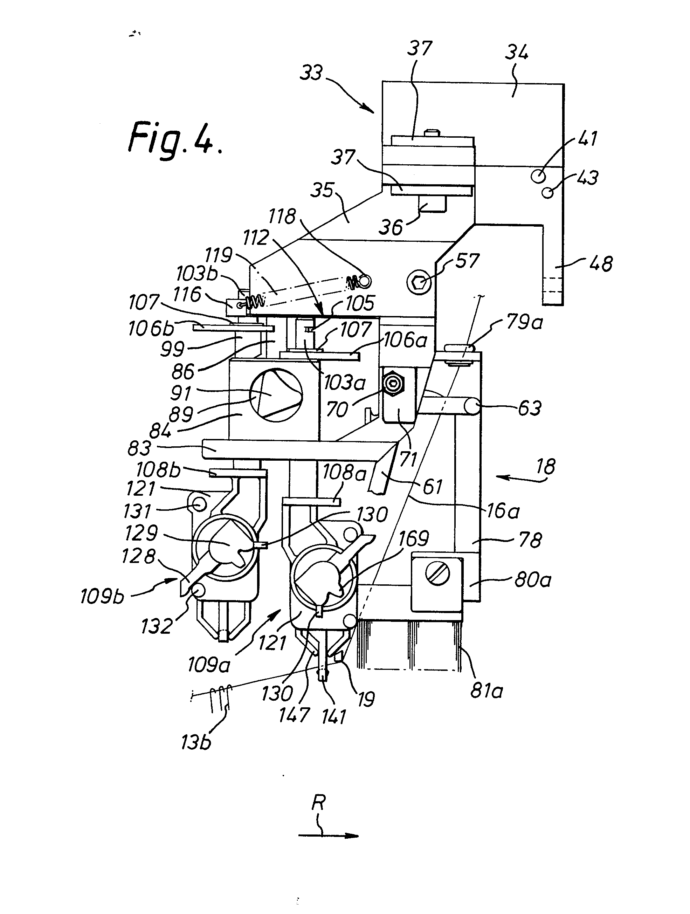

Der Arretierschieber 112 ist so ausgebildet und angeordnet,
daß die an den Ansätzen 103 angebrachten Quernuten 105, wenn
die beiden Kodierschieber 106 in einer Mittelstellung und
in gleicher Höhe angeordnet sind, in derselben Höhe wie
die Gabelarme 115 angeordnet sind (Fig. 2). Diese treten
daher beim Drehen der Trommel 94 in die Quernuten 105 ein,
wie in Fig. 8 durch die durchgezogene Stellung des Arretierschiebers 112 angedeutet ist. Dadurch sind die Zahnstangen 99 und mit ihnen die Fadenklemmen 109 gegen ungewollte Vertikalbewegungen verriegelt. Befindet sich dagegen
z.B. die Kodierscheibe 106b gemäß Fig. 4 und 6 in ihrer Hochstellung und daher die andere Kodierscheibe 106a in ihrer
Tiefstellung, dann kommt beim Verschwenken der Gabel nach
vorn das obere Ende des unten stehenden Ansatzes 103a dicht
unterhalb des Arretierschiebers 112 zu liegen, während der
andere Ansatz 103b in dem von den Gabelarmen 115 umgriffenen
Raum ragt und dadurch den Arretierschieber 112 etwas nach
der Seite drückt, wie in Fig. 8 durch die gestrichelte Lage
des Arretierschiebers 112 angedeutet ist. Daher ist bei dieser
Stellung eine unbeabsichtigte Vertikalverschiebung der Fadenklemmen 109 durch den auf dem Ansatz 103a aufliegenden Gabelarm 115 verriegelt.

Die Fadenklemmen 109 sind in Fig. 2 bis 6 in verschiedenen
Stellungen dargestellt. Sie enthalten eine im wesentlichen
vertikal nach unten ragende Montageplatte 121, die am zugehörigen Haltekörper 108 befestigt und gemäß Fig. 2 und 3
mit einer Mittelbohrung 122 (Fig. 2) versehen ist, in der
eine kreisrunde Lagerscheibe 123 drehbar gelagert ist. An
der aus der Mittelbohrung 122 herausragenden Vorderseite
dieser Scheibe 123 ist ein länglicher Verstellnocken 124 befestigt, an dessen Vorderseite eine Führungsscheibe 125
angebracht ist, die einen kleineren Querschnitt als die
Mittelbohrung 122 aufweist. Die Lagerscheibe 123 weist an ihrer Rückseite einen Flansch 126 auf, an dessen Rückseite ein Ansatz 127 mit einem Schaltfinger 128 angebracht ist, und an der Rückseite des Ansatzes 127 ist ein weiterer Ansatz 129 mit einem weiteren Schaltfinger 130 vorgesehen. Die Drehbewegung der Lagerscheibe 123 ist durch zwei mit dem Schaltfinger 128 zusammenwirkende Anschläge 131 und 132 begrenzt.

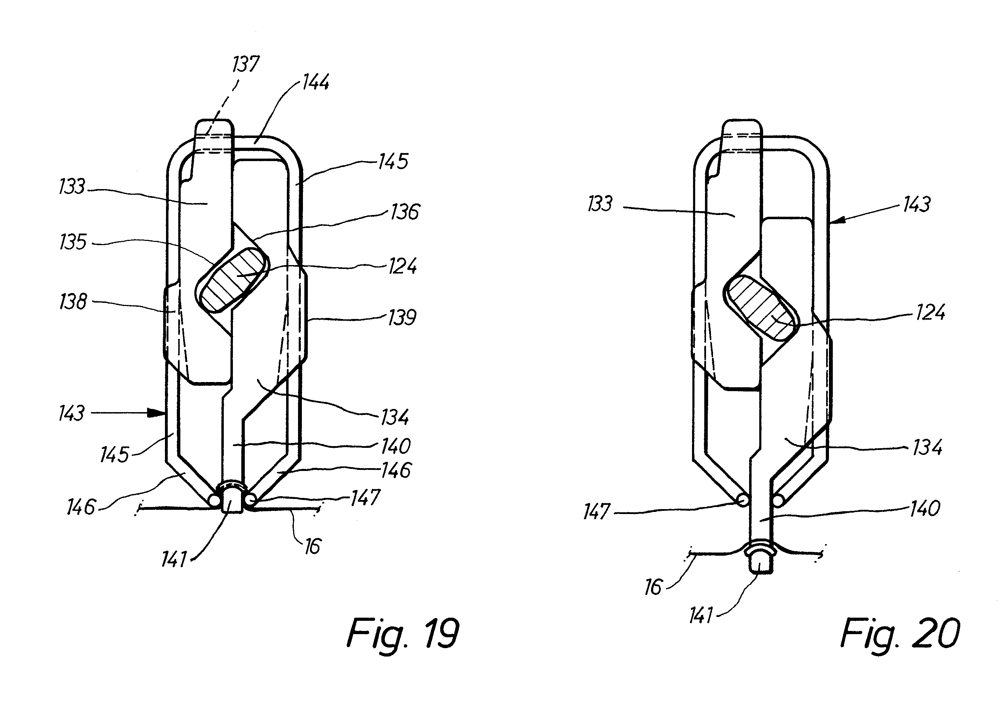

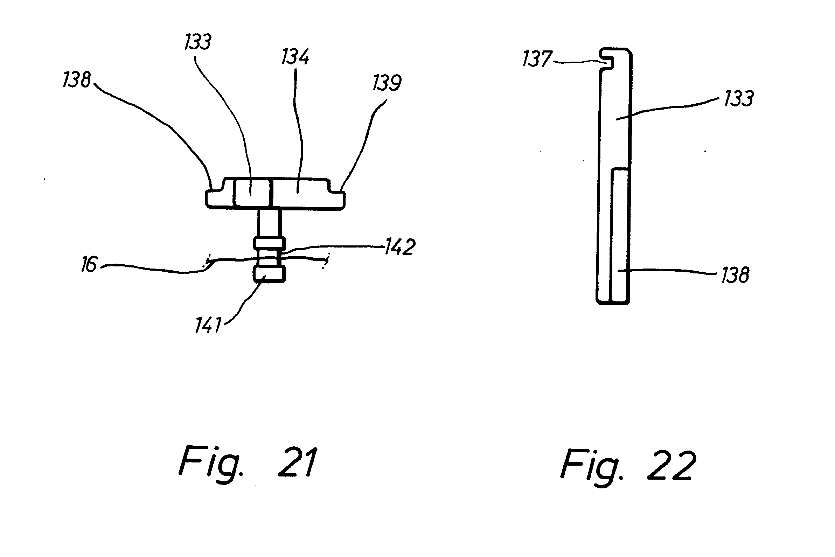

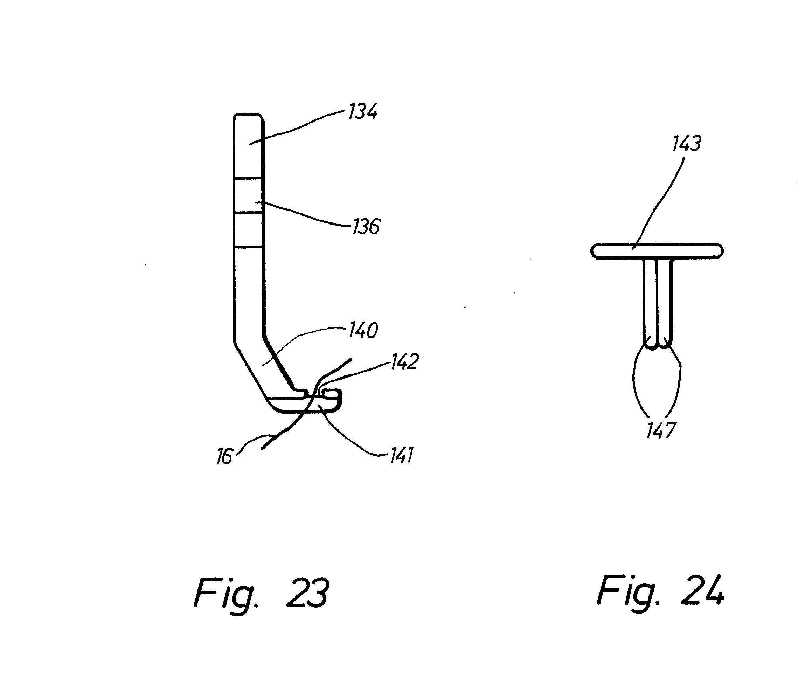

Nach dem Einsetzen der Lagerscheibe 123 in die Mittelbohrung 122, wobei sich der Flansch 136 der Rückseite der Montageplatte 121 anlegt, wird der Verstellnocken 124 in eine etwa 45°-Stellung gedreht. Sodann wird von beiden Seiten des Verstellnockens 124 her je ein flacher Schieber 133,134 mit im wesentlichen rechteckiger Grundform zwischen die Montageplatte 121 und die Führungsscheibe 125 geschoben. Beide Schieber weisen nach Fig. 19 bis 24 an einer Längsseite je eine dreieckförmige Aussparung 135,136 auf, in welcher der Verstellnocken 124 bei der Anordnung nach Fig. 19,20 gänzlich verschwindet, so daß die Schieber 133,134 mit ihren Längsseiten aneinander liegen. Der Schieber 133 ist an seinem oberen Rand mit einer rückwärtigen Quernut 137 und an seiner der Aussparung 135 gegenüberliegenden Längsseite mit einem rückwärtig angeformten Kragen 138 versehen. Der Schieber 134 ist an seiner der Aussparung 136 gegenüberliegenden Längsseite mit einem rückwärtig angeformten Kragen 139 und an seiner Unterseite mit einer schräg zu seinen Breitseiten angeordneten, nach vorn ragenden Verlängerung 140 versehen. Diese Verlängerung 140 trägt einen nach vorn ragenden, im wesentlichen horizontal angeordneten Klemmfinger 141, der in einem mittleren Teil eine Tasche 142 aufweist, in welcher der Faden beim Klemmvorgang zu liegen kommt (Fig. 21,23) und daher nicht nach vorn wegrutschen kann.

Die Fadenklemme 109 wird vervollständigt durch eine Klemmfeder 143, die nach Fig. 19,20 und 24 etwa rechteckförmig gebogen ist und von vorn so auf und hinter die beiden Schaltschieber 133,134 gedrückt wird, daß ihr oberer Quersteg 144 in der Quernut 137 und ihre beiden Längsstege 145 zwischen
den Kragen 138,139 und der Montageplatte 121 zu liegen kommen. An ihrem unteren Ende weist die Klemmfeder 143 zwei
trichterförmig aufeinander zulaufende Abschnitte 146 auf,
deren Enden um 90° nach vorn umgebogen sind und Klemmbacken
147 bilden. Diese Klemmbacken 147 sind elastisch gegeneinander vorgespannt und legen sich in der aus Fig. 19 ersichtlichen Klemmstellung von unten und parallel gegen den Klemmfinger 141, so daß ein dazwischen liegender Faden 16 sicher
eingeklemmt wird. Wird in dieser Klemmstellung der Verstellnocken 124 um ca. 90° im Uhrzeigersinn gedreht, dann wird
dadurch der Schieber 133 angehoben und der Schieber 134 gesenkt, so daß entsprechend die mit dem Schieber 133 gekoppelte Klemmfeder 143 und mit ihr die Klemmbakken 147 nach oben, der Klemmfinger 141 dagegen nach unten
in die Offenstellung der Fadenklemme 109 geschoben werden
(Fig. 20) und ein zuvor eingeklemmter Faden 16 frei gegeben
wird. Dabei bewirkt der Verstellnocken 124 beim Übergang von
der einen Einstellung in die andere durch seitliches Wegdrücken der Schieber 133,134 eine geringfügige Spreizung der Klemmfeder 143, wodurch den Klemmbacken 147 das
Überlaufen der durch die Tasche 142 gebildeten seitlichen
Vorsprünge erleichtert wird. Der in Fig. 19 gezeigten Endstellung des Verstellnockens 124 entspricht dabei das Anliegen des Schaltfingers 128 am unteren Anschlag 132, während
der in Fig. 20 gezeigten Endstellung des Verstellnockens 124
das Anliegen des Schaltfingers 128 am oberen Anschlag 131
entspricht. Die Klemmfeder 143 dient gleichzeitig zur
Sicherung der Lage des aus Lagerscheibe 123, Verstellnocken
124, Führungsscheibe 125, Flansch 126 und Ansätzen 127,129
gebildeten Bauelements auf der Montageplatte 121, ohne daß
weitere Befestigungselemente benötigt werden. Auf ihrer
Vorderseite ist die Montageplatte 121 zusätzlich mit zwei
Führungsnocken 148 versehen, die sich bei den Schaltvorgängen gegen die Längsstege 145 der Klemmfeder 143 legen
und diese seitlich führen. Im übrigen sind die Schieber 133,134
mit Gleitsitz zwischen der Montageplatte 121 und der Führungsscheibe 125 geführt.

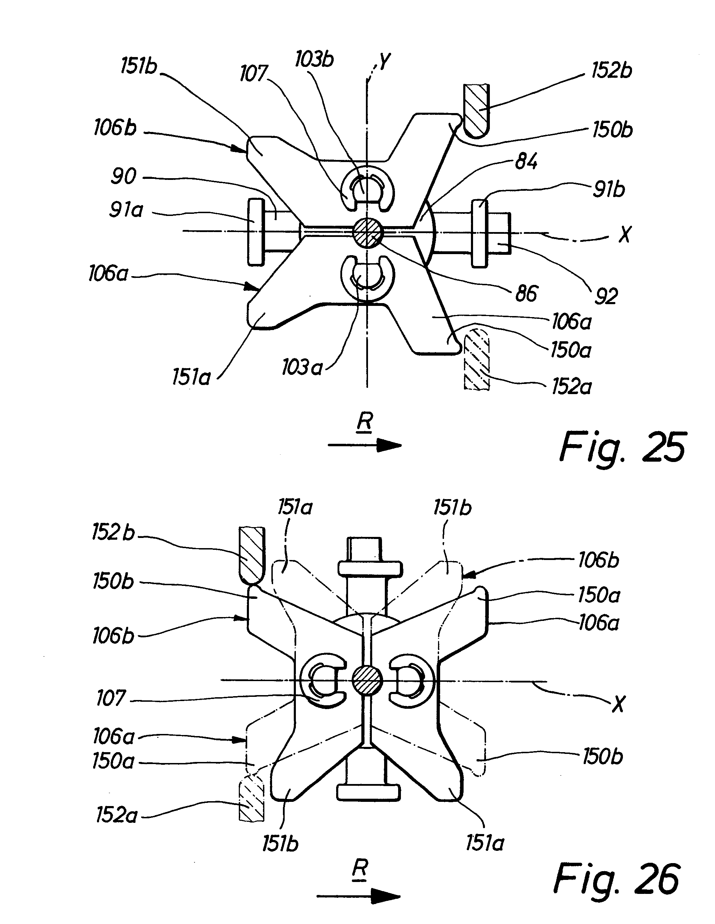

Die Ausbildung der Kodierscheiben 106a,b ist in Fig. 25,26
dargestellt. Sie bestehen aus flachen Scheiben, die an ihrer
Vorderseite und Rückseite je einen von der Mittelebene $\underline{X}$
des Fadenführers nach außen ragenden Lappen 150a,151a bzw.
150b,151b aufweisen. Dabei sind die Lappen 150 und 151b so
ausgebildet, daß in der Stellung nach Fig. 25 der maximale
Abstand der Lappen 150 von der Mittelebene $\underline{X}$ größer als der
entsprechende maximale Abstand der Lappen 151 ist, während
gleichzeitig der maximale Abstand der Lappen 150 von einer
durch die Drehachse gelegten, zur Mittelebene $\underline{X}$ senkrechten Ebene $\underline{Y}$ kleiner als der entsprechende maximale Abstand
der Lappen 151 ist. Die Abstandsdifferenz ist dabei so groß,
daß ein in den Weg der Kodierscheiben gebrachter Schaltnokken 152a,b durch Einwirkung auf einen vorderen Lappen 150a,b
die Kodierscheiben 106a,b um genau 90$^o$ in eine der beiden
Stellungen nach Fig. 26 (durchgezogen oder gestrichelt) dreht
bzw. daß der von einem dieser Nocken 152 beeinflußten Lappen 150 beim Abgleiten von diesen Nocken so steht (Fig. 26),
wie es einer genauen 90$^o$-Drehung der Kodierscheibe entspricht.
Entsprechendes gilt, wenn die Kodierscheiben 106 an einer
der beiden Stellungen nach Fig. 26 durch einen Schaltnocken,
der auf die dann weiter von der Mittelebene $\underline{X}$ beabstandeten
Lappen 151a,b einwirkt, um genau 90$^o$ zurück in d i e Stellung
nach Fig. 25 gedreht werden sollen.

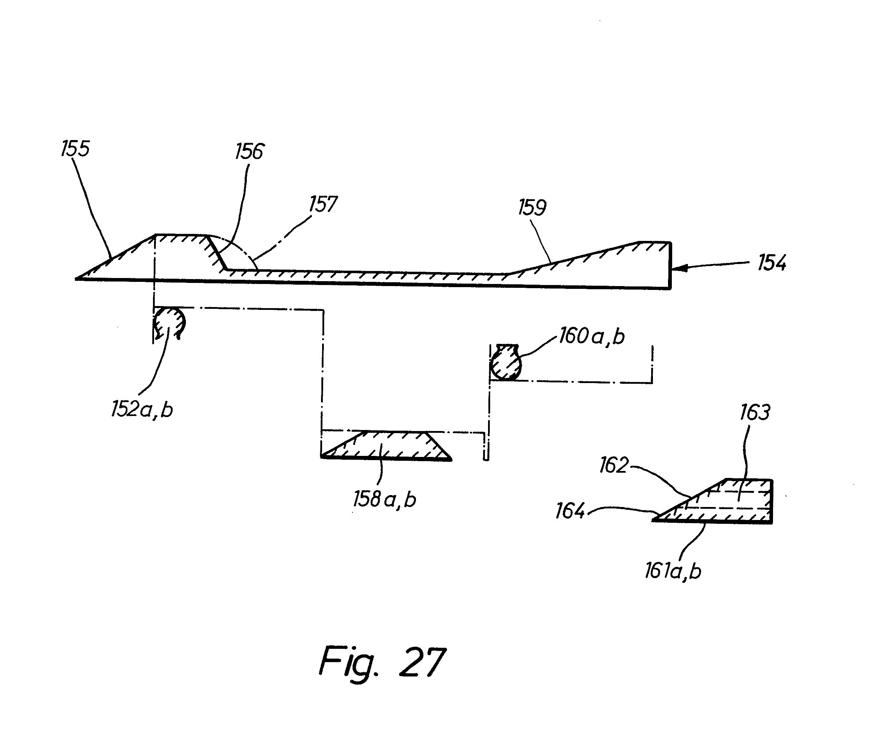

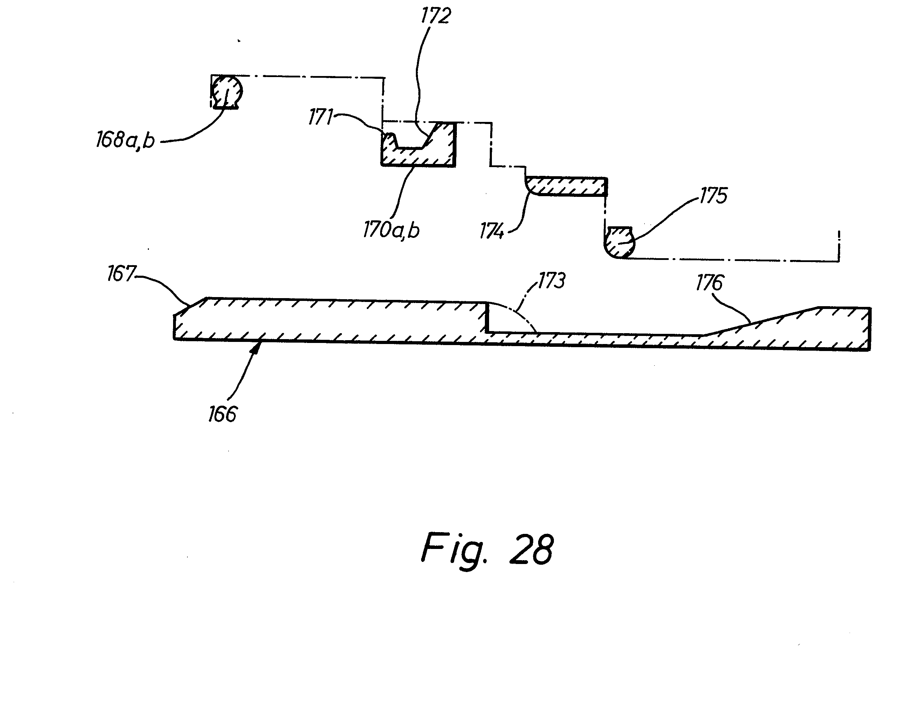

Zur automatischen Steuerung der mit dem Transportband 25
umlaufenden Fadenführer 18 sind am Beginn und am Ende des
jeweiligen Arbeitsabschnitts an sich bekannte Schaltstationen angeordnet (DE-OS 30 03 570), die Kurven und Nocken
aufweisen, mittels derer auf den Schwenkbügel 63, die Steuerscheiben 91, die Kodierscheiben 106 und/die Schaltfinger 128,
ggf. auch
130 eingewirkt wird und die zweckmäßig an der starren Schiene 28 oder einem damit verbundenen Bauteil befestigt sind.
Die benötigten Kurven und Nocken sind in Fig. 27 und 28
dargestellt.

Der beschriebene Fadenführer arbeitet wie folgt:

Jeder Fadenführer 18 nimmt vor dem Einlaufen in die am Anfang des Arbeitsabschnitts befindliche Schaltstation die
aus Fig. 2 ersichtliche Klemmstellung ein, in welcher die
Sperrnase 92 in der Aufnahme 93 liegt. Daher ist einerseits
der Steuerhebel 58 gegen die Kraft der Federn 119 festgelegt
und andererseits die Trommel 84 gegen Drehung gesichert.
Die Gabel 74 befindet sich in ihrer nach vorn geschwenkten
Stellung, während die Fadenklemmen 109 symmetrisch zu beiden
Seiten der Mittelebene des Fadenführers und mit nach vorn
gerichteten Klemmfingern 141 angeordnet ist.

Das nach rechts ragende freie Ende des Schwenkbügels 63
des Fadenführers läuft nun auf eine ortsfeste, in Fig. 27
in ihrer Seitenansicht dargestellte Kurve 154 mit einem
Hebeteil 155 auf, das den Steuerhebel 58 geringfügig derart
verschwenkt, daß die Aufnahme 93 abgesenkt und die Sperrnase 92
freigegeben wird. Soll mit keinem der beiden von einem Fadenführer mitgeführten Fäden gearbeitet werden, fällt der Schwenkbügel nach Verlassen der Kurve 154 unter dem Einfluß der Zugfeder 119 wieder in die Sperrstellung zurück.

Soll dagegen mit dem Fadenführer gestrickt werden, muß zunächst ausgewählt werden, welcher der beiden Fäden 16a,b
eingelegt werden soll. Diese Auswahl erfolgt mittels eines
der beiden Schaltnocken 152a bzw. 152b (Fig. 25,26), die zu
beiden Seiten der Mittelebene $\underline{X}$ in einer Tiefstellung angeordnet sind und mittels einer nicht dargestellten Steuervorrichtung, z.B. eines Hubmagneten, in eine Mittelstellung
gebracht werden können, in welcher sie in Höhe der in
Mittelstellung befindlichen Kodierscheiben 106a,b angeordnet
sind. Bei Auswahl des in Fig. 27 in der Draufsicht dargestellten Schaltnockens 152a läuft infolgedessen nur die
Kodierscheibe 106a auf diesen Schaltnocken auf, während
die andere Kodierscheibe 106b unbeeinflußt bleibt. Dies
hat zur Folge, daß sich die Trommel 84 beim Vorbeigang am
Schaltnocken 152a um 90$^{o}$ dreht und daher beide Fadenklemmen
109a,b hintereinander und links von der Mittelebene $\underline{X}$

angeordnet sind, wobei die Fadenklemme 109b vorausläuft.

Kurz nach Beginn der Trommeldrehung wird der Schwenkbügel
63 durch ein abfallendes Teil 156 der Kurve 154 freigegeben,
so daß der Steuerhebel 58 unter dem Einfluß der Zugfedern
119 entsprechend einer Linie 157 (Fig. 27) in die aus Fig. 7
ersichtliche Stellung geschwenkt wird, in welcher die Gabel 74 hinter dem von der Fadenklemme 109b gehaltenen
Faden 16b angeordnet ist. Der Faden 16a ist dabei außerhalb
des Wirkungsbereichs der Gabel 74 angeordnet.

Die infolge der Trommeldrehung quer zur Transportrichtung
angeordneten, mit ihren Spitzen nach unten ragenden, herzförmigen Steuerscheiben 91 gelangen nun in den Bereich von federnd gelagerten, von unten her angreifenden Hebekurven 158a,b
(Fig. 27) und werden von diesen in die aus Fig. 7 ersichtliche
Stellung gedreht. Da auf jeder Seite der Mittelebene $\underline{X}$ je eine
Hebekurve 158a,b angeordnet ist, kann sich dabei die Trommel
84 nicht ebenfalls drehen. Durch das Verdrehen der Steuerscheiben 91 werden die ausgewählte vordere Fadenklemme 109b und die
zugehörige Kodierscheibe 106b in die aus Fig. 7 ersichtlichen
Tiefstellungen bewegt, während gleichzeitig die andere Fadenklemme 109a und die andere Kodierscheibe 106a entsprechend in
Hochstellungen angehoben werden. Diese Verstellung der Fadenklemmen 109 ist zu diesem Zeitpunkt möglich, weil durch die
Verschwenkung des Steuerhebels 58 auch der Arretierschieber 112
so weit nach vorn gezogen wurde, daß er in keiner der Quernuten 105 der Ansätze 103 angeordnet ist. Dieselben Bewegungen
ergeben sich im Hinblick auf die Fadenklemme 109a, wenn statt
des Schaltnockens 152a der Schaltnocken 152b ausgewählt wird,
weil mittels der Hebekurven 158a,b stets diejenige Fadenklemme
abgesenkt wird, die nach der Drehung der Trommel 84 vorn steht.
In beiden Fällen befindet sich die Tasche 142 der abgesenkten
Fadenklemme und daher auch das in dieser befindliche Fadenende
in der Mittelebene $\underline{X}$ des Fadenführers, von wo aus der zugehörige
Faden 16 schräg nach oben zur zugehörigen Fadenöse 79 gespannt
ist, und unterhalb des Schwenkbereichs des Einlegeorgans 19.

- 19 -    0141955

Es sei nun angenommen, daß der Faden 16a zum Stricken ausgewählt wurde und daher die Fadenklemme 109a vorn steht. Der Fadenführer 18 gelangt nun in den Wirkungsbereich eines zweiten Hebeteils 159 der Kurve 154 und eines zweiten, ebenfalls in der Draufsicht dargestellten Nockens 160a,b für die Rückdrehung der Trommel 84. Dabei sind die Nocken 160a,b zu beiden Seiten der Mittelebene $\underline{X}$ in einer der Tiefstellung der Kodierscheiben 106 entsprechenden Höhe angeordnet, so daß nur der Nocken 160a wirksam wird. Die Lage des Hebeteils 159 und der Nocken 160a,b ist im übrigen so aufeinander abgestimmt, daß das Hebeteil 159 zunächst den Schwenkbügel 63 etwas anhebt, damit der Faden 16a von den beiden nach außen ragenden Gabelarmen der Gabel 74 erfaßt werden kann, und daß dann gleichzeitig die Trommel 84 zurückgedreht und der Schwenkbügel 63 weiter angehoben wird. Sobald die Drehung der Trommel vollendet ist, endet die Kurve 154, wodurch sich die Sperrnase 92 wieder in die Aufnahme 93 legt. Der Arretierschieber 112 ist jetzt entsprechend Fig. 4 angeordnet, so daß eine Auf- und Abbewegung der Zahnstangen 99 blockiert ist.

Beim Zurückschwenken des Steuerhebels 58 tritt der Faden 16a in die Gabel 74 ein. Dadurch wird einerseits das zwischen der Fadenklemme 109a und der Fadenöse 79a befindliche Fadenteil gespannt. Andererseits gleitet der Faden in der aus Fig. 3 ersichtlichen Weise in den nach vorn offenen, keilförmigen Kanal 76 des Einlegeorgans 19, der genau in Höhe des Arbeitsabschnitts angeordnet ist. Von dort ist der Faden bis zur Fadenklemme 109a quer zur Transportrichtung $\underline{R}$ gespannt (Fig. 5), so daß er in bekannter Weise (DE-OS 30 03 570) durch die erste ausgetriebene Nadel 13a des gegenüberliegenden Nadelbetts erfaßt und in die zum Einlegen in nachfolgende Nadeln richtige Position gebracht werden kann.

Nachdem der Faden 16a in die Nadeln 13 eingelegt ist, muß noch die Fadenklemme 109a geöffnet werden, um das Fadenende freizugeben. Hierzu weist die Schaltstation Hebeteile 161a,b auf, die in Fig. 27 als Seitenansicht und in Fig. 6 als Schnitt dargestellt und auf beiden Seiten der Mittelebene $\underline{X}$ angeordnet sind. Die Hebekurve 161a schwenkt den seitlich wegragenden Schaltfinger 128 der Fadenklemme 109a nach oben, wodurch deren Klemmfeder 143 nach oben, der Klemmfinger 141 dagegen nach unten bewegt und die Fadenklemme entsprechend Fig. 3 geöffnet wird.

Damit bei diesem Vorgang die andere Hebekurve 161b nicht auf die andere Fadenklemme 109b einwirkt, die geschlossen bleiben muß, weisen die Hebeteile 161a,b entsprechend Fig. 6 und 27 in ihrem mittleren Teil eine horizontale, bis zu einer Hebekurve 162 erstreckte Aussparung 163 auf. Die Hebeteile 161 sind dabei so bemessen, daß die freien Enden der Schaltfinger 128 der in Mittel- oder Hochstellung befindlichen Fadenführer 109 (in Fig. 6 rechts) in die Aussparung 162 eintreten und daher nicht angehoben werden. Das freie Ende des Schaltfingers 128 einer in Tiefstellung befindlichen Fadenklemme (in Fig. 6 links) läuft dagegen zunächst auf eine verbreiterte Spitze 164 der Hebekurve 162 auf und wird dadurch leicht verschwenkt. Da diese Verschwenkung mit einer Drehung um die Achse des Verstellnockens 124 verbunden ist, ragen die auf die Spitze 164 auflaufenden und dann einer Kreisbahn 165 folgenden Enden der Schaltfinger 128 beim Erreichen der Aussparung 163 radial weiter nach außen als bei den von der Spitze 164 nicht beeinflußten Schaltfingern 128, so daß sie dann die Aussparung 163 überbrücken und der Kurve 162 folgen. Damit ist das Einlegen des zugehörigen Fadens 16a beendet.

Ein besonderer Vorteil dieser Maßnahme besteht darin, daß mittels der Hebeteile 161 zwischen strickenden und nicht strickenden Fadenführern unterschieden werden kann und die Hebeteile nicht steuerbar sein brauchen, um das Öffnen solcher Fadenklemmen 109 zu vermeiden, die einen nicht strickenden Faden führen.

Die innerhalb des Arbeitsbereichs einen strickenden Faden führenden Fadenführer werden nach Verlassen des Arbeitsbereichs durch eine Schaltstation geführt, deren Kurven und Nocken in Fig. 2ª/Ansichten entsprechend Fig. 27 dargestellt sind. Im wesentlichen laufen dabei die bereits beschriebenen Arbeitsschritte in umgekehrter Reihenfolge ab.

Die dem strickenden F-den 16a zugeordnete Fadenklemme 109a
läuft in der aus Fig. 3 ersichtlichen Weise in die Schaltstation ein, wobei der strickende Faden 16a allerdings entsprechend Fig. 4 zwischen der letzten strickenden Nadel 13b
und dem Einlegeorgan 19 gespannt ist, das in Fig. 4 zur Vermeidung von Unklarheiten nur durch eine Umlenkstelle im
Fadenweg angedeutet ist.

Die Schaltstation weist zunächst eine Kurve 166 auf, die mit
einem ansteigenden Abschnitt 167 den Schwenkbügel 63 anhebt
und damit die Sperrnase 92 von der Aufnahme 93 löst. Sodann
gelangt der Fadenführer in den Bereich von zwei starren
Nocken 168a und b, die in der Höhe der in Hochstellung befindlichen Kodierscheiben 106 angeordnet sind und nur diese
beeinflussen. Infolgedessen wird beim beschriebenen
Beispiel nur die Kodierscheibe 106b vom Nocken 168b betätigt,
was eine Drehung der Trommel 84 um 90$^o$ in die aus Fig. 4 ersichtliche Position zur Folge hat. Dabei wird gleichzeitig
der Schwenkbügel 63 weiterhin oben gehalten. Dadurch sind
nach erfolgter Drehung der Trommel 84 die Klemmbacken 147
der vorderen Fadenklemme 109a oberhalb, der zugehörigen
Klemmfinger 141 dagegen unterhalb des zwischen der Nadel 13b
und dem Einlegeorgan 19 gehaltenen Fadenstücks angeordnet.
Als nächstes muß die Fadenklemme 109a geschlossen werden.
Da hierzu der Schaltfinger 128 wegen seiner Hochstellung
nicht benutzt werden kann, ist der zweite, in einer anderen
Ebene angeordnete Schaltfinger 130 so ausgerichtet, daß er
bei in Hochstellung befindlichem Schaltfinger 128 senkrecht
nach unten ragt. Da dieser Schaltfinger um ca. 90$^o$ verschwenkt werden muß, ist ihm zweckmäßig ein zweiter, radial
kürzerer und in derselb n Ebene liegender Schaltfinger 169
(Fig. 4) zugeordnet. Auf die Schaltfinger 130 und 169 können
zweistufige, auf beiden Seiten der Mittelebene X angeordnete
und entsprechend ausgebildete Kurven 170a,b (Fig. 28) einwirken.
Diese enthalten einen ersten Hebeabschnitt 171, der sich gegen
den Schaltfinger 130 legt und diesen um einen Teil seines Hubs
verschwenkt. Danach läuft der dann inzwischen weitergedrehte
und jetzt senkrecht nach unten ragende Schaltfinger 169 auf einen zweiten Hebeabschnitt 172 der Kurve 170 auf, wodurch die Fadenklemme 109a vollends geschlossen wird. Auf die jeweils in Hochstellung befindliche, nachlaufende Fadenklemme haben die Kurven 170 keinen Einfluß. In der aus Fig. 4 ersichtlichen Stellung der Fadenklemme 109a ist im übrigen die Tasche 142 des Klemmfingers 141 genau unter dem Faden angeordnet, so daß er bei der Schließbewegung in diese eintritt.

Nach dem Schließen der Fadenklemme 109a endet die Kurve 167 zunächst, so daß der Schwenkbügel 63 entsprechend einer Linie 173 durch die Zugfedern 119 verschwenkt wird und dadurch die Gabel 74 in die aus Fig. 7 ersichtliche Stellung gelangt, in der sie das zwischen der Fadenklemme 109a und der Fadenöse 79a befindliche Fadenstück freigibt. Anschließend wird das zwischen der Nadel 13b und der Fadenklemme befindliche Fadenstück auf an sich bekannte Art geschnitten. Parallel hierzu laufen die beiden Steuerscheiben 91 auf beidseitig der Mittelebene $\underline{X}$ und oberhalb ihrer Achsen angeordnete Kurven 174 auf, welche die Steuerscheiben 91 derart zurückdrehen, daß die beiden Kodierscheiben 106 wieder dieselbe Höhenlage, d.h. ihre Mittelstellung einnehmen. Schließlich gelangt der Fadenführer 18 in den Bereich von beidseitig angebrachten, starren Nocken 175. Diese wirken allerdings nicht auf die Kodierscheiben 106, sondern auf die beiden Haltekörper 108 ein, die an ihrem Umfang beispielsweise so wie die hinteren Lappen 151a,b ausgebildet sind, während die vorderen Lappen 150a,b fehlen, um Kollisionen mit dem jeweils inaktiven Nocken 175 zu vermeiden. Gleichzeitig wird mittels eines weiteren ansteigenden Abschnitts 176 der Kurve 166 der Schwenkbügel 63 angehoben, damit sich die Sperrnase 92 über die Aufnahme 93 legen kann und am Ende der Kurve 166 wieder die aus Fig. 2 ersichtliche, verriegelte Normalstellung des Fadenführers erreicht wird. Die Gabel 74 läuft dabei an dem von der zugehörigen Fadenklemme 109a nach außen gespannten Faden 16a vorbei, ohne ihn zu erfassen.

Die mit keinem ihrer Fäden strickenden Fadenführer könnten die am Ende des Arbeitsbereichs angeordnete Schaltstation nach Fig. 28 an sich unbeeinflußt durchlaufen. Dies hätte jedoch zur Folge, daß die quer zur Mittelebene $\underline{X}$ gemessene

Breite der Fadenführer in der aus Fig. 4 ersichtlichen Trommelstellung zumindest genau so groß wie die entsprechend gemessene Breite in der aus Fig. 2 ersichtlichen normalen Trommelstellung sein müßte, da andernfalls die in Normalstellung befindlichen Fadenführer nicht den Raum zwischen den beiden zum Schließen der Fadenklemmen benötigten Kurven 170 passieren könnte. Wegen der ohnehin beengten Raumverhältnisse an einer Strickmaschine ist jedoch erwünscht, dem Fadenführer in der Stellung nach Fig. 4 eine kleinere Breite als in der Stellung nach Fig. 2 zu geben.

Die Erfindung sieht daher vor, auch die nicht strickenden Fadenführer beim Passieren der Schaltstation nach Fig. 28 zu verschwenken. Dafür ist vorgesehen, auf der einen Seite der Mittelebene den Nocken 168, z.B. den Nocken 168b, so auszubilden, daß er nur die in Hochstellung befindlichen Kodierscheiben 106b erfassen kann, während der andere Nocken 168, z.B. der Nocken 168a, so angeordnet und ausgebildet ist, daß er sowohl in Hochstellung als auch in Mittelstellung einlaufende Kodierscheiben 106a beeinflußt. Dadurch werden die Trommeln 84 derjenigen Fadenführer, von denen ein Faden strickt, durch den entsprechenden, in Hochstellung wirksamen Nocken 168 gedreht, während der jeweils andere Nocken 168 wegen der tief stehenden anderen Kodierscheibe keinen Einfluß hat. Die Trommel 84 eines mit keinem Faden strickenden Fadenführers wird dagegen von dem bis zur Mittelstellung reichenden Nocken gedreht, da sich dessen beiden Kodierscheiben 106 in der Mittelstellung befinden.

Hinsichtlich der Rückdrehung dieser Fadenführer ist zu beachten, daß nach Passieren des Raums zwischen den Kurven 170 bei denjenigen Fadenführern, die einen strickenden Faden führen, noch die Gabeln 74 zurückgeschwenkt werden (Linie 173), bevor die Rückdrehung (Nocken 175) erfolgt. Diese Verschwenkung ist für die nicht strickenden Fadenführer unerwünscht, weil sich deren nach der Drehung vorn liegender Faden von außen gegen die noch nicht verschwenkte Gabel 74 legt und daher bei deren Verschwenkung von ihr erfaßt werden kann. Daher ist in der Schaltstation nach Fig. 28 ein weiterer, nicht gezeigter Nocken vorgesehen, der die Trommeln 84 der nicht strickenden Fadenführer nach deren Vorbeigang an den Kurven 170 sofort teilweise zurückdreht, um den Faden außerhalb des Wirkungsbereichs der Gabel anzuordnen. Dieser Nocken ist auf der erforderlichen Seite der Mittelebene X angeordnet und wirkt auf die in Mittelstellung befindlichen Kodierscheiben 206 der nicht strickenden Fadenführer ein. Die vollständige Rückdrehung der nicht strickenden Fadenführer wird dann wie bei den strickenden Fadenführern durch den Nocken 175 bewirkt, da erst bei dessen Erreichen die Aufnahme 93 für die Sperrnase 92 aus reichend abgesenkt ist.

Die Erfindung ist nicht auf das beschriebene Ausführungsbeispiel beschränkt. Dies gilt insbesondere für die Anordnung und Ausbildung der verschiedenen Teile der Fadenführer und der Schaltstationen, da in dieser Hinsicht dem Wunsch nach einer kleinen, raumsparenden Konstruktion Rechnung getragen wurde, um eine möglichst große Systemzahl an der Strickmaschine realisieren zu können.

Weiter ist es nicht erforderlich, den beschriebenen Fadenführer grundsätzlich mit zwei Fäden zu versehen. Alternativ kann jeder Fadenführer auch nur zur Zuführung eines einzigen Fadens vorgesehen werden, wobei entweder eine Fadenklemme unbesetzt bleibt oder alle für die Zuführung eines zweiten Fadens vorgesehenen Teile weggelassen werden. Auch bei dieser Anwendungsform kann der zugehörige Fadenführer wahlweise in den Kanal eingefädelt bzw. aus diesem herausgezogen werden, um dadurch strickende Fadenführer von nicht strickenden Fadenführern zu unterscheiden.

Schließlich können die auf die Schaltfinger 128 einwirkenden Hebeteile 161 längs der Nadelbetten verschiebbar und ggf. automatisch steuerbar angeordnet sein, damit sie beim Mindern bzw. Erweitern der Gestrickbreite individuelle auf die jeweils erste strickende Nadel eingestellt werden können.

Ansprüche
_____

1) Fadenführer für eine Strickmaschine mit einem Einlegeorgan
(19), das an seinem Auslaufende für den Faden (16) als offener Kanal (76) ausgebildet ist, und mit wenigstens einer steuerbaren Fadenklemme, dadurch gekennzeichnet, daß das Einlegeorgan (19) und die Fadenklemme (109) derart relativ zueinander
beweglich angeordnet sind, daß der Faden von der Fadenklemme
(109) selbst wahlweise in den Kanal (76) einlegbar oder aus
diesem herausnehmbar ist.

2) Fadenführer nach Anspruch 1, dadurch gekennzeichnet, daß
die Fadenklemme (109) an einer drehbaren Trommel (84) montiert ist.

3) Fadenführer nach Anspruch 2, dadurch gekennzeichnet, daß
die Trommel (84)  von einer quer zu ihrer Achse verlaufenden,
an wenigstens einem Ende mit einer Steuerscheibe (91) versehenen Welle (89) durchsetzt ist.

4) Fadenführer nach Anspruch 2, dadurch gekennzeichnet, daß
die Fadenklemme (109) an einer parallel zur Achse der Trommel (84) verschiebbar gelagerten Zahnstange (99) befestigt
ist.

5) Fadenführer nach Anspruch 4, dadurch gekennzeichnet, daß
die Zahnstange (99) wenigstens einen Zahn (101) aufweist,
der in eine an der Welle (89) ausgebildete Zahnlücke (97)
eingreift.

6) Fadenführer nach Anspruch 4, dadurch gekennzeichnet, daß
an einem aus der Trommel (84) herausragenden Ende der Zahnstange (99) eine Kodierscheibe (106) befestigt ist.

7) Fadenführer nach Anspruch 1, dadurch gekennzeichnet, daß
das Einlegeorgan (19) an einem Arm (61) eines auf einem Drehbolzen (66) schwenkbar gelagerten Steuerhebels (58) befestigt
ist.

8) Fadenführer nach Anspruch 6 und 7, dadurch gekennzeichnet, daß an dem mit der Kodierscheibe (106) versehenen Ende der Zahnstange (99) eine Quernut (105) ausgebildet ist und der Steuerhebel (58) einen zweiten Arm (60) aufweist, der an einem mit der Quernut (105) zusammenwirkenden, verschiebbar gelagerten und zur Verriegelung der Zahnstange (99) bestimmten Arretierschieber (112) angelenkt ist.

9) Fadenführer nach Anspruch 1, dadurch gekennzeichnet, daß die Fadenklemme (109) zwei durch einen Verstellnocken (124) in entgegengesetzt parallele Richtungen verschiebbare Schieber (133,134) aufweist, von denen der eine mit einem Klemmfinger (141) versehen und der andere mit einer Klemmfeder (143) gekoppelt ist, die wenigstens eine mit dem Klemmfinger (141) zusammenwirkende Klemmbacke (147) aufweist.

10) Fadenführer nach wenigstens einem der Ansprüche 5 bis 9, dadurch gekennzeichnet, daß er zwei gleichartig ausgebildete Fadenklemmen (109a,109b) aufweist, die symmetrisch auf beiden Seiten einer durch die Achse der Trommel (84) gelegten Ebene angeordnet und an je einer zur Trommelachse parallelen Zahnstange (99) befestigt sind, und daß jede Zahnstange (99) wenigstens je einen Zahn (101) aufweist, der in je eine von zwei sich diametral gegenüberliegenden Zahnlücken (97) der Welle (89) eingreift.

Fig.1.

0141955

2/18

Fig. 2

Fig.3.

Fig.4.

Fig.5.

Fig. 6.

Fig.7.

7/18

0141955

Fig. 8

Fig. 9

Fig. 10

Fig. 11

0141955

Fig. 12

Fig. 13

Fig. 14

11/18

Fig. 15          Fig. 16

Fig.17.

Fig.18.

Fig. 19

Fig. 20

14/18

Fig. 22

Fig. 21

*Fig. 23*

*Fig. 24*

Fig. 25

Fig. 26

*Fig. 27*

155  156  157  159  154  160a,b  152a,b  158a,b  162  163  164  161a,b

44/48

0141955

*Fig. 28*